# EUROPÄISCHE PATENTANMELDUNG

(11) **EP 3 120 814 A1**
(43) Veröffentlichungstag der Anmeldung: **25.01.2017**
(21) Anmeldenummer: 16001571.5
(22) Anmeldetag: 15.07.2016
(51) Int. Cl.: A61F 5/01

(54) **ORTHESE UND VERFAHREN ZUR HERSTELLUNG EINER SOLCHEN ORTHESE**

(30) Priorität: 21.07.2015 DE 102015009279
(71) Anmelder: Springer Aktiv AG, 13407 Berlin (DE)
(72) Erfinder: Hepper, Martin, 13465 Berlin (DE); Schreiter, Eric, 44135 Dortmund (DE)
(74) Vertreter: Sattler de Sousa e Brito, Clara

(57) **Zusammenfassung**

Eine Orthese mit einem Fußteil und einem mit dem Fußteil verbundenen Unterschenkelteil, ist dadurch gekennzeichnet, dass der Fußteil eine mit dem Unterschenkelteil verbundene Außenschale sowie eine innerhalb der Außenschale angeordnete und eine Negativform einer Fußsohle ausbildende Einlage (1) umfasst. Ein Verfahren zur Herstellung einer solchen Orthese umfasst zumindest folgende Verfahrensschritte:
- Erzeugung eines Sohlenabdrucks eines Fußes eines Patienten;
- Herstellung einer an den Sohlenabdruck angepassten Einlage (1);
- Erzeugung einer die Einlage (1) umfassenden Negativform des Fußes und des von der Orthese zu umfassenden Abschnitts des Unterschenkels;
- Erzeugung einer Positivform des Fußes und des von der Orthese zu umfassenden Abschnitts des Unterschenkels durch Ausfüllen der Negativform;
- Erzeugung des Unterschenkelteils und der Außenschale unter Nutzung der Positivform und der Einlage (1) als Formkern; und
- Einbringen der Einlage (1) in die Außenschale des Fußteils.

## Beschreibung

Die Erfindung betrifft eine Orthese mit einem Fußteil und einem Unterschenkelteil sowie ein Verfahren zur Herstellung einer solchen Orthese.

Eine Orthese ist ein medizinisches und konkret orthopädisches, einen Teil eines Körpers umgreifendes Hilfsmittel, das je nach medizinischer Indikation zur Stabilisierung, Entlastung, Ruhigstellung, Führung oder Korrektur von Gliedmaßen oder des Rumpfs eines Patienten eingesetzt wird. Eine Herstellung einer Orthese erfolgt auf ärztliche Verordnung oder auf privaten Wunsch hin individuell oder industriell vorgefertigt oder durch einen Orthopädietechniker beziehungsweise Orthopädieschuhtechniker.

Ein übliches Verfahren zur Herstellung einer Orthese mit einem Fußteil und einem Unterschenkelteil kann folgende Verfahrensschritte umfassen:
- Erzeugung eines Sohlenabdrucks, eines Blauabdrucks oder eines Scans eines Fußes eines Patienten. Dieser dient sowohl der noch folgenden Herstellung einer Orthese als auch einer gesetzlich vorgeschriebenen Dokumentation.
- Erzeugung eines sogenannten Fußbretts, das bei der sich anschließenden Erzeugung eines Gipsabdrucks zur Anwendung kommen soll. Hierzu ist das Fußbrett mit relativ groß dimensionierten Vertiefungen versehen, die der Aufnahme der prominenten Fußknochen im Bereich der Fußsohle dienen.
- Erzeugung eines Gipsnegatives durch zirkuläre Umwicklung des Beines durch Gipsbinden und oder Longuetten.
- Erzeugung einer Negativform des Fußes und des Abschnitts des Unterschenkels, der von der Orthese umfasst werden soll, aus Gips, wobei auf das Fußbrett zur Ausbildung einer Gipssohle sogenannte Gipspelotten aufgebracht werden, in denen sich die anatomische Sohlenform des Fußes exakt abdrückt. Die übrigen Abschnitte der Negativform werden dagegen mit Gipsbinden, die um den Fuß, das Fußbrett und den relevanten Abschnitt des Unterschenkels gewickelt werden, erzeugt.
- Erzeugung einer Positivform des Fußes und des relevanten Abschnitts des Unterschenkels durch Ausgießen der Negativform mit Gips. Durch die Verwendung einer Gipsisoliercreme wird dabei eine Verbindung zwischen der Negativform und der Positivform verhindert.
- Erzeugung der Orthese durch beispielsweise Tiefziehen von erwärmten Kunststoffplatten, wobei die Positivform als Formkern genutzt wird.
- Die Orthese kann dann noch hinsichtlich der äußeren Kantenform angepasst und mit Verschlusselementen, wie beispielsweise Klettbändern versehen werden.

Es ist ersichtlich, dass eine solche Herstellung einer Orthese relativ aufwändig ist. Dies gilt insbesondere für den Fußteil. Zudem ist ein Nachteil einer durch ein solches Verfahren hergestellten Orthese, dass die Sohlenform nach der Herstellung nicht oder nur unter einem großen Aufwand verändert werden kann. Dies ist jedoch in vielen Fällen erforderlich, weil es für das Wohlbefinden eines Nutzers einer solchen Orthese wichtig ist, dass die zeitweise mit einem Mehrfachen des Körpergewichts dynamisch belastete Sohle der Orthese möglichst exakt an die individuelle Form der Fußsohle des Nutzers angepasst ist, um Druckstellen zu vermeiden. Nachbesserungen sind in dieser Hinsicht häufig erforderlich, insbesondere bei Wachstum gerade bei Kinderversorgungen.

Ausgehend von diesem Stand der Technik lag der Erfindung die Aufgabe zugrunde, eine verbesserte Orthese, die einen Fußteil und einen Unterschenkelteil aufweist, anzugeben. Insbesondere sollte die verbesserte Orthese eine einfache Herstellbarkeit und/oder eine einfache Anpassung der Sohleninnenseite nach der Herstellung ermöglichen.

Diese Aufgabe wird mittels einer Orthese gemäß dem Patentanspruch 1 sowie mittels eines Verfahrens zur Herstellung einer solchen Orthese gemäß dem Patentanspruch 8 gelöst. Vorteilhafte Ausgestaltungen einer solchen Orthese und vorteilhafte Ausführungsformen des erfindungsgemäßen Herstellungsverfahrens sind Gegenstände der weiteren Patentansprüche und ergeben sich aus der nachfolgenden Beschreibung der Erfindung.

Eine gattungsgemäße Orthese mit einem Fußteil und einem mit dem Fußteil verbundenen Unterschenkelteil ist erfindungsgemäß dadurch gekennzeichnet, dass der Fußteil eine mit dem Unterschenkelteil verbundene Außenschale sowie eine innerhalb der Außenschale angeordnete und eine Negativform einer Fußsohle ausbildende Einlage (die somit als separates Element ausgebildet ist), umfasst.

Ein Verfahren zur Herstellung einer solchen Orthese kann vorteilhafterweise folgende Verfahrensschritte umfassen:
- Erzeugung eines Sohlenabdrucks eines Fußes eines Patienten.
- Herstellung einer an den Sohlenabdruck angepassten Einlage. Dies kann beispielsweise spanabhebend und insbesondere durch Fräsen von zumindest der die Negativformgebung zu dem Sohlenabdruck ausbildenden Seite erfolgen.
- Erzeugung einer die Einlage umfassenden Negativform des Fußes sowie des von der Orthese zu umfassenden Abschnitts des Unterschenkels. Anders als bei dem bekannten Verfahren wird bei einem erfindungsgemäßen Verfahren der Sohlenabdruck somit nicht nur zur Diagnose und/oder zu Dokumentationszwecken sondern maßgeblich zur Ausbildung der Sohle beziehungsweise zumindest der Sohleninnenseite der Orthese genutzt. Auf diese Weise werden die besonders aufwändigen Verfahrensschritte bei dem konventionellen Verfahren, die der Erzeugung einer individualisierten Gipssohle dienen (einschließlich der Erzeugung des Fußbretts) vermieden.
- Erzeugung einer Positivform des Fußes und des von der Orthese zu umfassenden Abschnitts des Unterschenkels durch Ausfüllen der Negativform. Dies kann insbesondere durch Ausgießen mit einem fließfähigen, aushärtbaren Werkstoff, wie beispielsweise Gips erfolgen.
- Erzeugung des Unterschenkelteils und der Außenschale unter Nutzung der Positivform (nach ihrer Aushärtung) und der Einlage als (zweiteiliger) Formkern. Dies kann vorzugsweise durch Tiefziehen eines plastisch verformbaren und anschließend aushärtbaren Rohlings, beispielsweise einer erwärmten Platte oder eines Blocks aus Kunststoff (z.B. PE, PP, PET, PPTA, CFK und/oder GFK) erfolgen. Ebenso kann eine Erzeugung des Unterschenkelteils und der Außenschale durch das Auflegen und Aushärten lassen von Prepregs, d.h. von mit Reaktionsharzen vorimprägnierten textilen Halbzeugen, die unter Temperatur- und/oder Druckbeeinflussung ausgehärtet werden, erfolgen. Eine Kombination beider Herstellungsvarianten für den Unterschenkelteil und die Außenschale ist ebenfalls möglich, so dass beispielsweise Prepregs außenseitig als zusätzliche Verstärkung auf einen durch Tiefziehen hergestellten Grundkörper (mit vorzugsweise relativ geringer Wandstärke) aufgebracht werden können.
- Einbringen der Einlage in die Außenschale des Fußteils. Demnach wird die Einlage nicht nur für die Erzeugung eines den Unterschenkelteil und die Außenschale des Fußteils umfassenden Schalenkörpers genutzt, sondern diese stellt auch einen Teil der Orthese selbst dar.

Infolge der genannten Merkmale und Vorteile ist das erfindungsgemäße Herstellungsverfahren weniger aufwändig.

In einer bevorzugten Ausgestaltung einer erfindungsgemäßen Orthese kann vorgesehen sein, dass die Einlage lösbar in der Außenschale angeordnet ist. Ergänzend zu dem erfindungsgemäßen Verfahren kann dazu vorgesehen werden, dass während oder nach dem Einbringen der Einlage in die Außenschale des Fußteils eine lösbare Verbindung zwischen diesen Komponenten ausgebildet wird. Ein besonders relevanter Vorteil einer lösbaren Anordnung der Einlage in der Außenschale des Fußteils im Vergleich zu einer ebenfalls möglichen nicht (zerstörungsfrei) lösbaren Anordnung liegt darin, dass nach der Herstellung und insbesondere nach einer bereits erfolgten Nutzung (gegebenenfalls nach einer ersten Anprobe und/oder einer Einlaufphase) durch einen Nutzer eine nachträgliche Anpassung der Einlage, insbesondere hinsichtlich der Form der Sohleninnenseite, erfolgen kann. Diese nachträgliche Anpassung ist infolge der dann von dem Rest der Orthese separierten Handhabung der Einlage vergleichsweise einfach.

Ein weiterer besonderer Vorteil ist, durch die Möglichkeit einer manuellen Herausnahme der Einlage aus der Orthese, kann diese gesäubert und gereinigt werden. Der hygienische Aspekt wird deutlich verbessert.

Dementsprechend kann im Rahmen eines erfindungsgemäßen Verfahrens vorteilhafterweise noch vorgesehen sein, dass die Einlage nach einer Anprobe der Orthese durch den vorgesehenen Nutzer aus dem Fußteil entnommen und separat nachbearbeitet wird. Ein weiterer Vorteil ist, dass die Einlage separat (d.h. ohne den Rest der Orthese; zum Beispiel in einem Schuh) genutzt werden kann, wenn der Heilungsprozess erfolgreich verlaufen ist und der Arzt diese alternativ verordnet.

In einer bevorzugten Ausgestaltung der erfindungsgemäßen Orthese kann vorgesehen sein, dass die Einlage in zumindest einem Abschnitt und insbesondere in zumindest einer Schicht, die sich vorzugsweise über die gesamte Hauptfläche der Einlage erstreckt, aus einem Material ausgebildet ist, das bis zu einer Temperatur von mindestens 180°C, vorzugsweise bis zu einer Temperatur von mindestens 200°C und besonders bevorzugt bis zu einer Temperatur von mindestens 220°C formstabil ist. Dadurch kann insbesondere verhindert werden, dass sich eine Formveränderung der Einlage bei der Herstellung der Orthese im Rahmen eines erfindungsgemäßen Verfahrens einstellt. Dies gilt insbesondere für eine Erzeugung des Unterschenkelteils und der Außenschale des Fußteils durch Tiefziehen, bei dem die Einlage einer entsprechend erhöhten Temperatur ausgesetzt sein kann. Aber auch bei einem Aushärten von Prepregs können relativ hohe Temperaturen auftreten (z.B. Aushärtung bei 130°C über einen Zeitraum von ca. drei Stunden oder bei 80°C über einen Zeitraum von ca. fünf Stunden).

Besonders bevorzugt kann vorgesehen sein, dass die Einlage in zumindest einem Abschnitt und insbesondere in zumindest einer Schicht, die sich vorzugsweise über die gesamte Hauptfläche der Einlage erstreckt, aus einem Mischmaterial, das Kork (insbesondere aus der Rinde der Korkeiche) aufweist, ausgebildet ist. Vorteile der diesbezüglichen Verwendung von Kork sind neben einer ausreichenden thermischen Formstabilität, was insbesondere die Herstellbarkeit im Rahmen eines erfindungsgemäßen Verfahrens positiv beeinflusst, eine hohe Elastizität, durch die Stöße bei der Nutzung der Orthese abgefedert werden, eine gute Luftdurchlässigkeit, die eine Transpiration in der Orthese verringert, sowie eine geringe Wärmeleitfähigkeit, die ebenfalls das Wohlbefinden bei der Nutzung der Orthese positiv beeinflussen kann, da ein Auskühlen des Fußes bei einer Nutzung bei geringen Außentemperaturen vermieden werden kann.

Weiterhin besonders bevorzugt kann vorgesehen sein, dass die Einlage in zumindest einem Abschnitt aus einem Mischmaterial, das neben Kork, Calciumcarbonat (Kalk) und/oder Kaolin und/oder Ethylenvinylacetat und/oder Polyethylen niedriger Dichte (PE-LD) insbesondere Bralen umfasst. Kaolin kann dabei insbesondere eine Flexibilität der Einlage positiv beeinflussen während Ethylenvinylacetat und Bralen einen positiven Einfluss auf die Elastizität aufweisen kann. Calciumcarbonat kann sich dagegen positiv auf die thermische Formstabilität des Mischmaterials beziehungsweise der daraus ausgebildeten Einlage auswirken.

Weiterhin bevorzugt kann vorgesehen sein, dass der Abschnitt aus dem thermisch formstabilen Material und insbesondere aus dem Mischmatieral, das Kork und/oder die weiteren Bestandteile aufweist, auf der der Negativform der Fußsohle abgewandten Seite mit einer Stützschicht aus einem relativ (verglichen mit dem thermisch formstabilen Material und insbesondere dem Mischmaterial das Kork und/oder die weiteren Bestandtele aufweist) steifen Material kombiniert ist. Dabei kann sich die Stützschicht ebenfalls vorzugsweise über die gesamte Hauptfläche der Einlage erstrecken. Die Stützschicht kann dazu dienen, die Einlage insbesondere bei dem Verfahrensschritt der Erzeugung einer die Einlage umfassenden Negativform des Fußes und des von der Orthese zu umfassenden Abschnitts des Unterschenkels zu stabilisieren, wodurch die Qualität der erzeugten Negativform positiv beeinflusst werden kann.

Weiterhin kann vorteilhafterweise der Abschnitt aus dem thermisch formstabilen Material und insbesondere aus dem Mischmatieral, das Kork und/oder die weiteren Bestandteile aufweist auf der die Negativform der Fußsohle ausbildenden Seite mit einer Funktionsschicht, beispielsweise aus einem Polstermaterial und/oder einem eine Transpiration hemmenden Material kombiniert sein. Eine solche Funktionsschicht kann insbesondere das Wohlbefinden eines Nutzers bei einer Nutzung der Orthese verbessern. Im Rahmen eines erfindungsgemäßen Verfahrens zur Herstellung einer erfindungsgemäßen Orthese kann dazu vorgesehen sein, dass die Polsterschicht frühestens nach der Erzeugung des Unterschenkelteils und der Außenschale des Fußteils unter Nutzung der Positivform und der Einlage als Formkern auf den Abschnitt und insbesondere die Schicht aus thermisch formstabilem Material und insbesondere aus dem Mischmatieral, das Kork und/oder die weiteren Bestandteile aufweist aufgebracht wird. Dies kann insbesondere dann vorteilhaft sein, wenn das Material der Funktionsschicht hinsichtlich der Temperaturen, denen die Einlage im Rahmen dieses Verfahrensschritts ausgesetzt ist, nicht beständig ist.

Der Unterschenkelteil und/oder die Außenschale des Fußteils können vorteilhafterweise aus PE (Polyethylen), PP (Polypropylen) und/oder PET (Polyethylenterephthalat) ausgebildet ist. Vorteilhaft möglich kann auch die Verwendung von CFK (mit Carbonfasern verstärkter Kunststoff), von GFK (mit Glasfasern verstärkter Kunststoff) und von PPTA (Aramid), insbesondere in Form von mit PPTA-Fasern verstärktem Kunststoff, sein. Dies kann ermöglichen, den Unterschenkelteil und/oder die Außenschale des Fußteils mit relativ geringen Wandstärken auszubilden, ohne dass dies mit einer Instabilität der Orthese einhergeht. Dadurch kann insbesondere das Gewicht der Orthese gering gehalten werden.

Vorzugsweise kann es sich um eine Einlage mit propriozeptiver Wirkungsweise handeln, d.h. hypertone Muskel werden durch Verlängerung der Strecke zwischen Ansatz und Ursprung gehemmt und hypotone Muskel werden durch Verkürzung der Strecke zwischen Ansatz und Ursprung aktiviert. Insbesondere kann der Zehensteg und die Retro Pelotte die Plantarflexoren Muskelgruppe (Achillessehne) hemmen, das laterale Längsgewölbe den M. fibularis longus/ M fibularis brevis aktivieren und das mediale Längsgewölbe den M. tibialis anterior aktivieren.

Alternativ kann es sich auch um einer Einlage mit einer Wirkungsweise handeln wie sie erstmals von Nancy Hylton beschrieben wurde. Hier wird eine biomechanische Stabilisierung des Fußes durch Aufrichtung des Steigbügels erreicht, die afferenten Nervenbahnen können eine bessere Tiefensensibilität durch die Kompression des Fußes weiter an das zentrale Nervensystem geben, der Stabilitätsgewinn erzeugt eine Tonusreduktion, der Fuß wird in reflexarmer Stellung gehalten und es gibt eine begrenzte Freigabe im oberen Sprunggelenk. Besonders von Vorteil ist wenn die Rückfußpunkte der Fersenstütze an der Ferseninnen- und - außenseite die gleiche Höhe aufweisen.

Dabei werden die Vorteile insbesondere bei einheitlicher Betrachtung von Orthese, Einlage und Fuß sichtbar. So kann durch die Kompression durch die PP-Schale eine bessere Tiefensensibilität erreicht werden. Durch eine korrigierte Form der PP- Schale kann sowohl eine Aufrichtung des Steigbügels (in Verbindung mit Längsgewölbe Modulen) als auch ein Stabilitätsgewinn erzeugt werden, der zu einer Tonusreduktion beziehungsweise zu einer reflexarmen Stellung führt. Durch eine Tieferlegung des großen Zehenballen kann der Vorfuß in eine zusätzliche Pronationsstellung gebettet werden, was zu einer besseren Bettung und Druckverteilung führt. Eine bessere Kontrolle des Bewegungsablaufs ist zudem durch Rückfußstabilisierung mittels Unterstützung im medialen und lateralen Längsgewölbe möglich.

Die unbestimmten Artikel ("ein", "eine", "einer" und "eines"), insbesondere in den Patentansprüchen und in der die Patentansprüche allgemein erläuternden Beschreibung, sind als solche und nicht als Zahlwörter zu verstehen. Entsprechend damit konkretisierte Komponenten sind somit so zu verstehen, dass diese mindestens einmal vorhanden sind und mehrfach vorhanden sein können.

Die Erfindung wird nachfolgend im Rahmen der Beschreibung konkreter Ausführungsformen erfindungsgemäßer Verfahren unter Verwendung von Zeichnungen näher erläutert. Die Zeichnungen zeigen dabei die durch ein solches Verfahren hergestellte Orthese beziehungsweise einzelne Bestandteile davon. Konkret zeigen die Zeichnungen in der
- Fig. 1:: eine Einlage einer erfindungsgemäßen Orthese;
- Fig. 2:: ein aus dem Unterschenkelteil und der Außenschale des Fußteils einer erfindungsgemäßen Orthese bestehender Schalenkörper;
- Fig. 3:: den Schalenkörper gemäß der Fig. 2 mit zusätzlich integrierten Polsterungen und Verschlusselementen; und
- Fig. 4:: eine erfindungsgemäße Orthese.

In einer Ausführungsform eines erfindungsgemäßen Verfahrens wird zunächst auf der Basis einer Anamnese eines Patienten ein Bestellbogen ausgefüllt, wobei unter anderem Angaben zur Indikation, zum äußeren Erscheinungsbild und maßbedingte Angaben zu Fertigung angegeben werden.

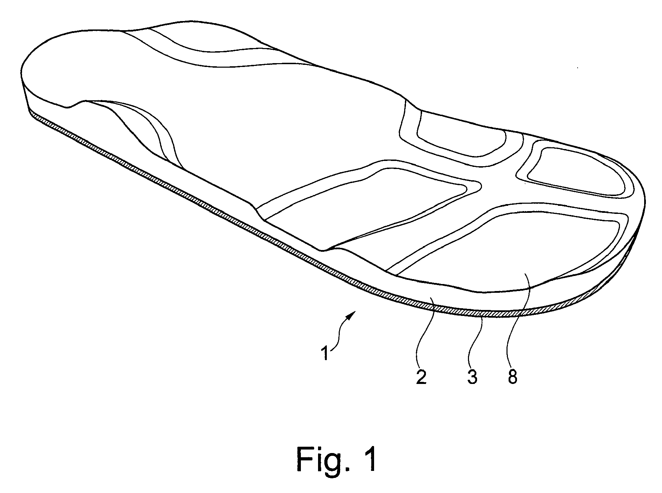

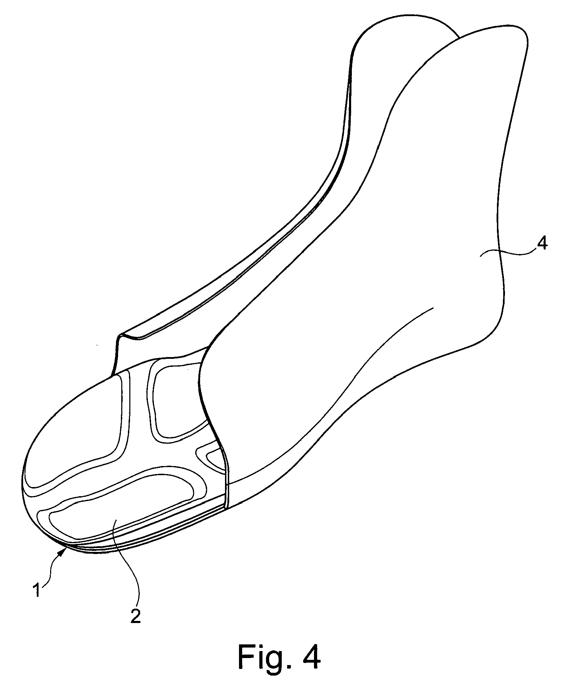

Weiterhin wird ein Sohlenabdruck desjenigen Fußes des Patienten, für den die Orthese hergestellt werden soll, gemacht. Dies kann beispielsweise mittels eines Schaumabdrucks, eines 2D- oder 3D-Scans oder auch eines Blauabdrucks erfolgen. Der Sohlenabdruck dient zum einen einer gesetzlich geforderten Dokumentation und zum anderen der Herstellung einer an den Sohlenabdruck angepassten Einlage 1 (vgl. Fig. 1 und 4).

Die Herstellung dieser Einlage 1 beziehungsweise zumindest einer Hauptschicht davon kann beispielsweise durch Fräsen aus einem Materialrohling aus einem ausreichend thermisch formstabilen Material, beispielsweise aus dem Mischmatieral, das Kork und/oder die weiteren Bestandteile aufweist, erfolgen. Hierbei kann eine CNC-Fräse zum Einsatz kommen, die eine kostengünstige und exakte Erzeugung der Einlage 1 in kurzer Zeit ermöglichen kann. Eine Reproduzierbarkeit ist gegeben.

Die Einlage 1 beziehungsweise die Hauptschicht 2 der Einlage 1 aus dem thermisch formstabilen Material kann mit einer Stützschicht 3 kombiniert werden, die beispielsweise eine Stärke von ungefähr 2 mm aufweisen und beispielsweise aus PP ausgebildet sein kann. Die Stützschicht 3 kann dabei mit der Hauptschicht 2 fest verbunden oder unverbunden sein. Die Stützschicht 3 dient der Stabilisierung der Einlage 1 beim nachfolgenden Erzeugen einer die Einlage 1 umfassenden Negativform des Fußes und des von der Orthese zu umfassenden Abschnitts des Unterschenkels mittels eines Gipsabdrucks.

Anschließend wird eine solche die Einlage 1 umfassende Negativform des Fußes mittels eines Gipsabdrucks erzeugt. Dies kann zunächst eine Kontrolle der Modulpositionierung, des Tonus und der Stellung der Einlage 1 am Fuß umfassen. Sofern erforderlich, kann die Einlage 1 nachbearbeitet werden, wobei insbesondere die Breite der Einlage 1 an den Fuß angepasst werden kann. Dabei kann besonders relevant sein, dass die Einlage 1 im Bereich der Ferse nicht zu schmal ausfällt, um für eine nachträgliche Befestigung einer Fersenkappe geeignet zu sein. Sofern erforderlich, kann auch eine Korrektur des Oberflächenprofils der Einlage 1 vorgenommen werden, wobei gegebenenfalls Korrekturkeile zum Einsatz kommen können. Daraufhin erfolgt ein Einfetten des Fußes und des relevanten Abschnitts des Unterschenkels mit einer Gipsisoliercreme, die ein zu starkes Anhaften des Gipses an der Haut verhindert.

Anschließend wird zunächst die Fersenkappe als Teil der Negativform ausgebildet. Hierzu wird der Fuß auf die Einlage 1 gestellt und mit einem länglichen Gipsstück der Calcaneus vom Längsgewölbe bis zum Peronealbogen umschlossen.

Daraufhin wird der Fuß mit beispielsweise Fixierband (z.B. Klebeband) an der mit der Fersenkappe verbundenen Einlage 1 fixiert. Dies erfolgt in Abhängigkeit von der zuvor ermittelten medizinischen Indikation: bei einem Knickfuß und/oder einem abgesenkten Hohlfuß wird primär eine Supinationszugrichtung vorgesehen, während bei einem Hohlfuß und/oder einem Klumpfuß eher eine Pronationszugrichtung vorgesehen wird. Weiterhin können prominente Stellen und insbesondere die Knöchel mit beispielsweise einem Kopierstift markiert werden.

Anschließend werden zur Erzeugung der Negativform gewässerte Gipsbinden um den Fuß und die unter dem Fuß befindliche Einlage 1 sowie um den relevanten Abschnitt des Unterschenkels gewickelt. Dabei kann es sinnvoll sein, eine gehemmte Stellung der Gelenke in einem Winkel von ca. 90° während des Anbringens der Gipsbinden zu beachten.

Nach einem Aushärten der Gipsbinden, was ca. fünf Minuten in Anspruch nehmen kann, kann die Negativform von dem Fuß und dem Unterschenkel des Patienten entfernt werden.

Anschließend kann die die Einlage 1 umfassende Negativform bearbeitet werden, wobei insbesondere das zuvor an der Einlage 1 für eine Fixierung an dem Fuß angebrachte Fixierband entfernt und auf der Innenseite der Negativform eine Gipsisoliercreme aufgetragen werden kann.

Daraufhin wird die Negativform zur Erzeugung einer Positivform des Fußes und des von der Orthese zu umfassenden Abschnitts des Unterschenkels mit Gips ausgegossen. Nach dem Austrocknen kann diese Positivform aus Gips von der Negativform einschließlich der Einlage 1 getrennt werden.

Anschließend können Modellierungsarbeiten durchgeführt werden. Dabei können insbesondere der Unterschenkelteil und das Fußbett der Positivform auf Maß gebracht und die Form der Einlage 1 angepasst werden. Weiterhin können Umfangs- und Weitemaße kontrolliert werden.

Ein darauffolgender Verfahrensschritt stellt die Erzeugung des Unterschenkelteils und der Außenschale des Fußteils unter Nutzung der Positivform und der Einlage 1 als Formkern dar. Die Erzeugung diese integralen Schalenkörpers 4 erfolgt durch Tiefziehen aus einer erwärmten Kunststoffplatte. Für eine Orthese für ein Kind kann beispielsweise eine 2 mm oder 3 mm starke Kunststoffplatte aus PP und für eine Orthese für einen Erwachsenen beispielsweise eine 5 mm starke Kunststoffplatte aus einem thermoplastischen Copolymer (PETG), beispielsweise dem von der SIMONA AG unter dem Handelsnamen SIMLOUX vertriebenen Kunststoff, verwendet werden. Hierzu wird zunächst die Einlage 1 gesäubert. Gegebenenfalls können auch noch eine Spannlasche 5 und Knöchelpolster 6 an die Positivform aus Gips angepasst werden. Weiterhin kann auch die Breite der Einlage 1 weiter angepasst werden. Hierzu kann ein Brandsohlenzuschliff vorgesehen werden, bei dem durch ein Abschleifen der Einlagenbrandsohle (planare Seite der Einlage) eine größere Flexibilität gegenüber zu hohem Druck in der Orthese durch beispielweise ein Wachstum des Fußes oder einer inexakten Umfangsmodellierung des Gipses gewährleistet wird.

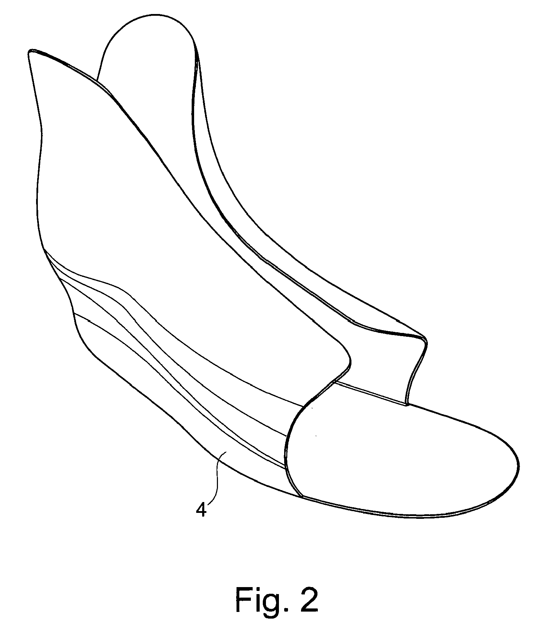

Anschließend kann vorgesehen sein, einen sogenannten "Dummy" zu laminieren, der für die spätere Herstellung einer separaten Socke und für ein Aufschneiden der noch herzustellenden Schaleneinheit 4 der Orthese (vgl. Fig. 2 bis 4) genutzt werden kann.

Daraufhin erfolgt das Tiefziehenhermoformen, wobei die erwärmte Kunststoffplatte unter plastischer Verformung um die Einheit aus Positivform, Einlage 1 und gegebenenfalls die Spannlasche 5 (bzw. einem entsprechenden ausreichend thermisch formstabilen Dummy) sowie die Knöchelpolster 6 (bzw. entsprechenden ausreichend thermisch formstabilen Dummies) geformt wird. Mögliche Verfahrenstemperaturen können dabei zwischen 160°C und 180°C bei der Verwendung von PE für die Schaleneinheit 4, zwischen 180°C und 200°C bei der Verwendung von PP für die Schaleneinheit 4 und zwischen 200°C und 220°C bei der Verwendung von PET für die Schaleneinheit 4 liegen.

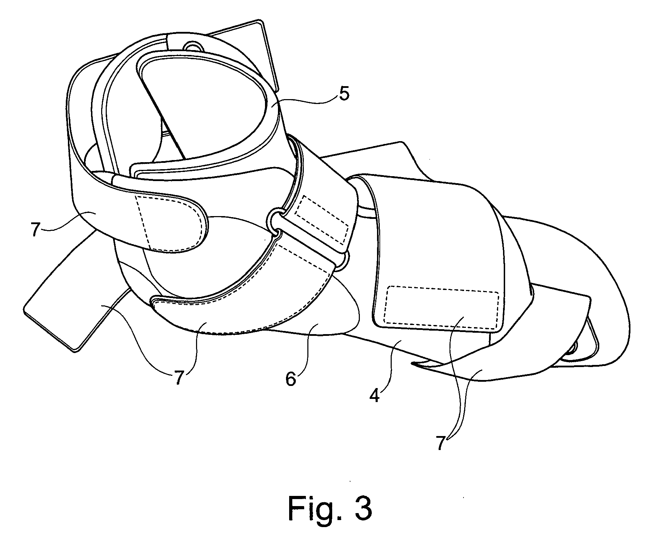

Anschließend kann die äußere Kantenform der den Unterschenkelteil und die Außenschale des Fußteils umfassende Schaleneinheit 4 angepasst sowie beschliffen werden. Die Fig. 2 zeigt eine entsprechend ausgebildete Schaleneinheit 4. Weiterhin können Verschlusselemente 7 provisorisch daran befestigt werden. Für die darauf folgende Anprobe können zudem die Einlage 2, die Spannlasche 5 sowie die Knöchelpolster 6 provisorisch an der Schaleneinheit 4 fixiert werden (vgl. Fig. 3, jedoch ohne Einlage 1).

Bei der Anprobe wird die Modulpositionierung der Einlage 1 und dessen Funktionsweise, das Volumen der gesamten Orthese, der Fersensitz und der Fersenhalt, insbesondere hinsichtlich möglicher Druckstellen, der Sitz der Verschlusselemente 7 und die Kantenform der Orthese kontrolliert und gegebenenfalls angepasst.

Daraufhin kann eine dynamische Laufanalyse des Patienten mit angelegter Orthese durchgeführt werden. Anhand des Ergebnisses dieser Laufanalyse kann durch insbesondere eine Formveränderung der Einlage 1 die Hemmung und die Stabilität des Fußes und/oder des von der Orthese umfassten Abschnitts des Unterschenkels verändert und optimiert werden. Durch die Möglichkeit, die Einlage 1 aus der Schaleneinheit 4 zu entfernen, ist eine solche Nachbearbeitung der Einlage 1 einfach möglich. Weiterhin kann vorgesehen sein, die Positivform aus Gips zu bearbeiten, um Druckstellen oder Stellungsfehler zu korrigieren.

Nach der Anprobe werden die gewünschten Veränderungen vorgenommen. Daraufhin werden die Knöchelpolster 6, die Spannlasche 5 und gegebenenfalls Weichpolsterpelotten eingeklebt, die Verschlusselemente 7 vernäht und an die Schaleneinheit 4 genietet oder mit dieser verklebt.

Zudem kann vorgesehen sein, die Einlage 1 zu beziehen, beispielsweise auf der die Negativform der Fußsohle ausbildenden Seite mit einer Funktionsschicht 8, wodurch der Tragekomfort der Orthese erhöht werden kann. Die Fig. 1 zeigt eine solche Einlage 1 in isolierter Darstellung. Gegebenenfalls kann es sinnvoll sein, vor dem Beziehen eine Einlaufphase von beispielsweise ein bis zwei Monaten vorzusehen, um eine Nachbearbeitbarkeit der Einlage 1 infolge der bereits fest in die Einlage 1 integrierten Funktionsschicht 8 nicht zu erschweren.)

Daraufhin kann Einlage 1 in der Außenschale des Fußteils der Orthese fixiert werden (vgl. Fig. 4). Dies kann beispielsweise unter Verwendung von Kontaktkleber, Klettband in Verbindung mit Flauschband, oder doppelseitigem Klebeband erfolgen.

Nach der Komplettierung der Orthese kann diese je nach Bedarf von dem Patienten getragen werden. Bei Bedarf oder im Rahmen von Kontrollterminen kann die Einlage 1 aus der Orthese genommen werden, wodurch eine gute Kontrolle (beispielsweise bei einem Wachstum des Patienten oder bei Druckstellen) und eine sehr gute individuelle Nachbearbeitungsmöglichkeit gewährleistet wird.

Ein weiterer besonderer Vorteil ist, durch die Möglichkeit einer manuellen Herausnahme der Einlage aus der Orthese, kann diese gesäubert und gereinigt werden. Der Hygienische Aspekt wird deutlich verbessert.

In einer Abwandlung des zuvor beschrieben Verfahrens kann vorgesehen sein, die Schaleneinheit 4 (wie beschrieben) mit (einer) relativ dünnen Wandstärke(n) auszubilden. Diese dient dann als Grundkörper, der nachträglich mit einer aus Prepregs ausgebildeten Verstärkungsschicht verstärkt wird, um insbesondere eine ausreichende Stabilität zu erreichen.

Hierzu kann vorgesehen sein, dass die Einheit aus Grundkörper der Schaleneinheit 4 und darin aufgenommener Positivform, Einlage 1, gegebenenfalls Spannlasche 5 (bzw. entsprechender Dummy), sowie gegebenenfalls Knöchelpolster 6 (bzw. entsprechende Dummies) mit Folie in Unterdruck gebracht, daraufhin der Grundkörper außenseitig mit beispielsweise Carbonfasern und/oder Aramidfasern aufweisende Prepregs laminiert wird. Dabei können gegebenenfalls Gelenke in die Prepreg-Laminierung eingearbeitet werden. Anschließend kann eine Abdichtung durch eine zweite Folie vorgesehen werden. Anschließend kann ein Unterdruck zwischen den zwei Folien und damit in der Laminierungsschicht erzeugt werden. Daraufhin werden die Prepregs ausgehärtet. Dies kann beispielsweise durch ein Erhitzen unter Unterdruck bei einer Temperatur von ca. 120°C oder ca. 130°C über einen Zeitraum von ca. drei Stunden oder bei einer Temperatur von ca. 80°C über einen Zeitraum von ca. fünf Stunden erfolgen.

### Bezugszeichenliste

- 1: Einlage
- 2: Hauptschicht der Einlage
- 3: Stützschicht der Einlage
- 4: Schaleneinheit
- 5: Spannlasche
- 6: Knöchelpolster
- 7: Verschlusselement
- 8: Funktionsschicht der Einlage

## Patentansprüche

1. Orthese mit einem Fußteil und einem mit dem Fußteil verbundenen Unterschenkelteil, **dadurch gekennzeichnet, dass** der Fußteil eine mit dem Unterschenkelteil verbundene Außenschale sowie eine innerhalb der Außenschale angeordnete und eine Negativform einer Fußsohle ausbildende Einlage (1) umfasst.

2. Orthese gemäß Anspruch 1, **dadurch gekennzeichnet, dass** die Einlage (1) lösbar in der Außenschale angeordnet ist.

3. Orthese gemäß Anspruch 1 oder 2, **dadurch gekefnnzeichnet,** dass die Einlage (1) in zumindest einem Abschnitt aus einem Material ausgebildet ist, dass bis zu einer Temperatur von mindestens 180°C, vorzugsweise bis zu einer Temperatur von mindestens 200°C und besonders bevorzugt bis zu einer Temperatur von mindestens 220°C formstabil ist.

4. Orthese gemäß einem der vorhergehenden Ansprüche, **dadurch gekennzeichnet, dass** die Einlage (1) in zumindest einem Abschnitt aus einem Mischmaterial, das Kork und/oder, Calciumcarbonat und/oder Kaolin und/oder Ethylenvinylacetat und/oder Polyethylen niedriger Dichte aufweistt, ausgebildet ist.

5. Orthese gemäß Anspruch 3 oder 4, **dadurch gekennzeichnet, dass** der Abschnitt aus dem thermisch formstabilen Material oder aus dem Mischmaterial auf der der Negativform der Fußsohle abgewandten Seite mit einer Stützschicht (3) aus einem relativ steifen Material kombiniert ist.

6. Orthese gemäß einem der Ansprüche 3 bis 5, **dadurch gekennzeichnet, dass** der Abschnitt aus dem thermisch formstabilen Material oder aus dem Mischmaterial auf der die Negativform der Fußsohle ausbildenden Seite mit einer Funktionsschicht (8) kombiniert ist.

7. Orthese gemäß einem der vorhergehenden Ansprüche, **dadurch gekennzeichnet, dass** der Unterschenkelteil und/oder die fAußenschale des Fußteils aus PE, PP und/oder PET ausgebildet ist.

8. Verfahren zur Herstellung einer Orthese gemäß einem der vorhergehenden Ansprüche, **gekennzeichnet durch:**
- Erzeugung eines Sohlenabdrucks eines Fußes eines Patienten;
- Herstellung einer an den Sohlenabdruck angepassten Einlage (1);
- Erzeugung einer die Einlage (1) umfassenden Negativform des Fußes und des von der Orthese zu umfassenden Abschnitts des Unterschenkels;
- Erzeugung einer Positivform des Fußes und des von der Orthese zu umfassenden Abschnitts des Unterschenkels **durch** Ausfüllen der Negativform;
- Erzeugung des Unterschenkelteils und der Außenschale unter Nutzung der Positivform und der Einlage (1) als Formkern; und
- Einbringen der Einlage (1) in die Außenschale des Fußteils.

9. Verfahren gemäß Anspruch 8, **dadurch gekennzeichnet, dass** die Einlage nach einer Anprobe der Orthese aus dem Fußteil entnommen und separat nachbearbeitet wird.

10. Verfahren gemäß Anspruch 8 oder 9, **dadurch gekennzeichnet, dass** der Unterschenkelteil und die Außenschale des Fußteils durch Tiefziehen erzeugt werden.
